# DEMANDE DE BREVET EUROPEEN

(11) **EP 1 923 013 A1**
(43) Date de publication de la demande: **21.05.2008**
(21) Numéro de dépôt: 07356163.1
(22) Date de dépôt: 19.11.2007
(51) Int. Cl.: A61B 17/80

(54) **Dispositif prothetique ou d'osteosynthese a olive fendue**

(30) Priorité: 20.11.2006 FR 0610141
(71) Demandeur: TORNIER, 38330 Saint-Ismier (FR)
(72) Inventeur: Manceau, Thierry, 38400 Saint Martin D'heres (FR)
(74) Mandataire: Grand, Guillaume

(57) **Abrégé**

Ce dispositif comporte au moins une olive (20) de liaison mécanique entre un corps prothétique ou d'ostéosynthèse (11) et une vis d'ancrage osseux (30). Cette olive est adaptée, lors du vissage de la tête de vis (32) dans un orifice intérieur (24) de l'olive, pour passer, par écartement relatif des bords d'une fente (25) de l'olive, d'une configuration, dans laquelle l'olive est logée de manière mobile dans un trou traversant (15) du corps, tout en étant bloquée en rotation autour de son axe (Y-Y) par un moyen de blocage (40) solidaire du corps et s'étendant au moins partiellement à l'intérieur du trou de manière à être reçu entre les bords de la fente de l'olive, à une configuration dans laquelle l'olive relie rigidement la vis et le corps par coincement entre la tête de vis et une paroi (18A) du trou. Suivant une réalisation peu onéreuse et facile à fabriquer, le moyen de blocage comporte un pion (40) rapporté de manière fixe dans un logement (19) délimité par le corps et débouchant dans le trou, le pion occupant de manière continue le logement et une partie du trou.

## Description

La présente invention concerne un dispositif prothétique ou d'ostéosynthèse, destiné à être implanté au niveau d'un site osseux à stabiliser, ainsi qu'un procédé de fabrication d'un tel dispositif. L'invention concerne ainsi en particulier des plaques prothétiques ou d'ostéosynthèse destinées à réunir des morceaux d'os fragmentés, par exemple à la suite d'une fracture, appartenant à un os long, notamment à un humérus. De telles plaques permettent de réduire une fracture du col anatomique de l'humérus ou de l'extrémité de l'humérus située du côté du coude, ou bien une fracture de l'extrémité supérieure du tibia, voire d'autres lésions épiphysaires d'os longs.

Ce genre de plaque, dont un exemple est donné dans WO-A-2003/007832, est fixé à l'os par des vis qui comportent chacune, d'une part, une tige filetée d'ancrage osseux, introduite à travers un trou traversant de la plaque, et, d'autre part, une tête filetée d'immobilisation de la vis par rapport à la plaque, vissée dans la paroi du trou taraudée de façon complémentaire. En pratique, la direction d'ancrage des vis n'est pas toujours alignée avec l'axe de révolution de la paroi taraudée du trou de la plaque, si bien qu'il est nécessaire d'interposer, entre la tête de vis et la paroi du trou, une olive destinée à accommoder le désalignement entre l'axe de la vis et l'axe du trou. L'olive est extérieurement conformée pour, tant que la tête de vis n'est pas vissée dans l'olive, être mobile contre une paroi lisse du trou à la façon d'une rotule, tandis que, lorsque la tête de vis est progressivement vissée dans l'olive, cette dernière se déforme pour s'appuyer fermement contre la paroi du trou jusqu'à lier rigidement par coincement la vis et la plaque. En pratique, pour pouvoir être déformée, l'olive est fendue de part en part de sorte que les bords de la fente correspondante s'écartent l'un de l'autre lors du vissage de la tête de vis. Un exemple en est donné dans DE-U-200 22 673.

A l'usage, ces olives sont délicates à manipuler : lors du vissage de la tête dans l'olive, le taraudage de cette dernière est difficilement mis en prise avec le filetage de la tête de vis car l'olive a tendance à tourner sur elle-même dans le trou de la plaque en raison de la liaison encore mobile entre l'olive et la paroi de ce trou. Le chirurgien a ainsi des difficultés à visser efficacement la tête de vis dans l'olive, ce qui complique les gestes chirurgicaux et allonge le temps d'intervention.

Pour remédier à cet inconvénient, on a proposé, alors que la tête de vis n'est pas encore engagée dans l'olive, de bloquer l'olive en rotation autour de l'axe de son orifice, par rapport au trou du corps prothétique ou d'ostéosynthèse, grâce à un renflement saillant de la face extérieure de l'olive, qui est reçu dans une rainure creusée dans la paroi du trou. Ce renflement vient butter dans l'une ou l'autre des extrémités de la rainure lorsque l'olive tend à tourner autour de l'axe de son orifice, ce qui immobilise l'olive en rotation autour de cet axe, sans l'empêcher de basculer dans le trou pour accommoder un éventuel désalignement entre l'axe du trou et la direction d'ancrage de la vis choisie par le chirurgien. Un exemple de ce genre d'olive fendue à face extérieure localement renflée est illustré sur les figures 15 à 20 du document US-A-2005/0154392.

En pratique, la réalisation d'un tel renflement de blocage en rotation pose des problèmes de fabrication car la présence de ce renflement ne doit pas nuire aux caractéristiques géométriques du reste de la face extérieure de l'olive, par exemple à sa sphéricité, faute de quoi l'olive tend à se coincer dans le trou dans des positions non souhaitées. Pour atteindre un niveau de fiabilité acceptable, les coûts de fabrication sont alors importants.

Une autre solution permettant de bloquer l'olive en rotation est proposée par US-A-2005/085913, qui évoque, sans les représenter, un pion ou un renflement saillant, situé à l'intérieur du trou de réception de l'olive et prévu pour s'étendre dans la fente de l'olive. US-A-2005/085913 ne donne cependant aucun détail de réalisation correspondant, ne fournissant ainsi aucune solution pratique et économique réelle.

Le but de la présente invention est de proposer un dispositif à olive fendue amélioré, dans lequel l'olive est, avant écartement de sa fente, bloquée en rotation autour de son axe principal par un moyen facile à fabriquer, efficace et peu onéreux.

A cet effet, l'invention a pour objet un dispositif prothétique ou d'ostéosynthèse, tel que défini à la revendication 1.

Le pion de blocage du dispositif conforme à l'invention peut coopérer avec l'un des bords de la fente de l'olive pour bloquer cette dernière en rotation autour de son axe principal, l'empêchant ainsi de tourner sur elle-même à l'intérieur du trou traversant du corps. Ainsi, contrairement aux dispositifs de l'art antérieur dont les moyen de blocage de l'olive nécessitent des aménagements, à la fois, au niveau de la paroi du trou et au niveau de la face extérieure de l'olive, le moyen de blocage selon l'invention met à profit la présence de la fente de l'olive, de sorte qu'aucun aménagement additionnel n'est nécessaire au niveau de l'olive. En pratique, seul le corps prothétique ou d'osthéosynthèse est à aménager, de manière à solidariser à ce dernier le pion de blocage selon l'invention grâce au logement prévu à cet effet, ce qui est particulièrement facile à fabriquer et peu onéreux. En outre, comme ce pion comble le logement, la structure du corps n'est pas fragilisée de manière significative.

Des caractéristiques avantageuses de ce dispositif, prises isolément ou suivant toutes les combinaisons techniquement possibles, sont définies aux revendications dépendantes 2 à 7.

L'invention a également pour objet un procédé de fabrication d'un dispositif prothétique ou d'ostéosynthèse, tel que défini à la revendication 8.

Le procédé selon l'invention permet de fabriquer le dispositif tel que défini ci-dessus.

Des caractéristiques avantageuses de ce procédé sont définies aux revendications 9 à 11.

L'invention sera mieux comprise à la lecture de la description qui va suivre, donnée uniquement à titre d'exemple et faite en se référant aux dessins sur lesquels :
- la figure 1 est une vue en perspective de l'extrémité supérieure d'un humérus, sur lequel est implantée une plaque d'ostéosynthèse appartenant à un dispositif selon l'invention ;
- la figure 2 est une coupe partielle suivant le plan Il de la figure 1, représentant, en éclaté, la plaque humérale ainsi que des pions, une olive et une vis du dispositif ;
- la figure 3 est une vue prise suivant la flèche III à la figure 2, illustrant l'un des pions, l'olive et une partie de la plaque du dispositif ;
- la figure 4 est une vue analogue à la figure 1, illustrant, de manière partielle et en éclaté, une variante du dispositif conforme à l'invention ; et
- la figure 5 est une coupe partielle suivant le plan V de la figure 4, représentant, en éclaté, les composants de la figure 4, ainsi qu'une olive et une vis du dispositif.

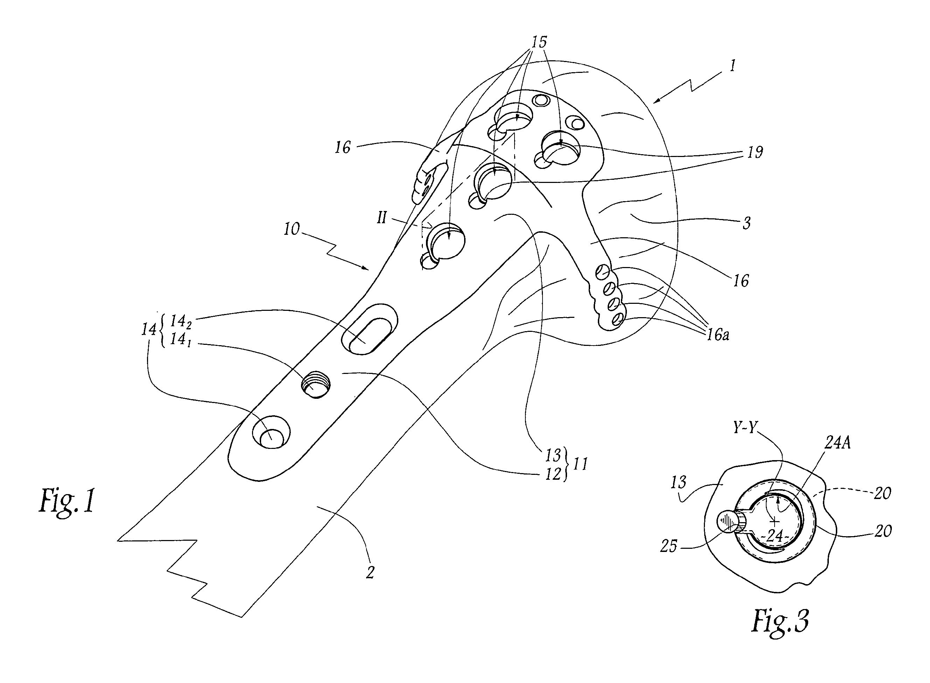

Sur la figure 1 est schématiquement représentée la partie d'extrémité supérieure d'un humérus 1 dont la diaphyse et l'épiphyse sont respectivement référencées 2 et 3. Par commodité, la suite de la description sera orientée par rapport à l'humérus dans sa position anatomique pour un patient se tenant debout, de sorte que les termes « supérieur » et « haut » désignent une direction dirigée vers le haut sur les figures 1 à 3, tandis que les termes « inférieur » et « bas » désignent une direction opposée.

Sur l'humérus 1 est mise en place une plaque humérale 10 comportant un corps principal allongé 11 s'étendant suivant la direction longitudinale de l'humérus, à la fois au niveau de sa diaphyse 2 et de son épiphyse 3. Le corps 11 inclut ainsi une partie diaphysaire 12 et une partie épiphysaire 13 situées respectivement au niveau de la diaphyse et de l'épiphyse de l'humérus.

La partie 12 est traversée, dans son épaisseur, de plusieurs trous 14 débouchant sur la diaphyse humérale 2, dont un trou taraudé 14₁ et un trou de section oblongue 14₂. Ces trous 14 ne seront pas décrits plus avant, étant noté que leur nombre et/ou leur géométrie ne sont en rien limitatifs de l'invention.

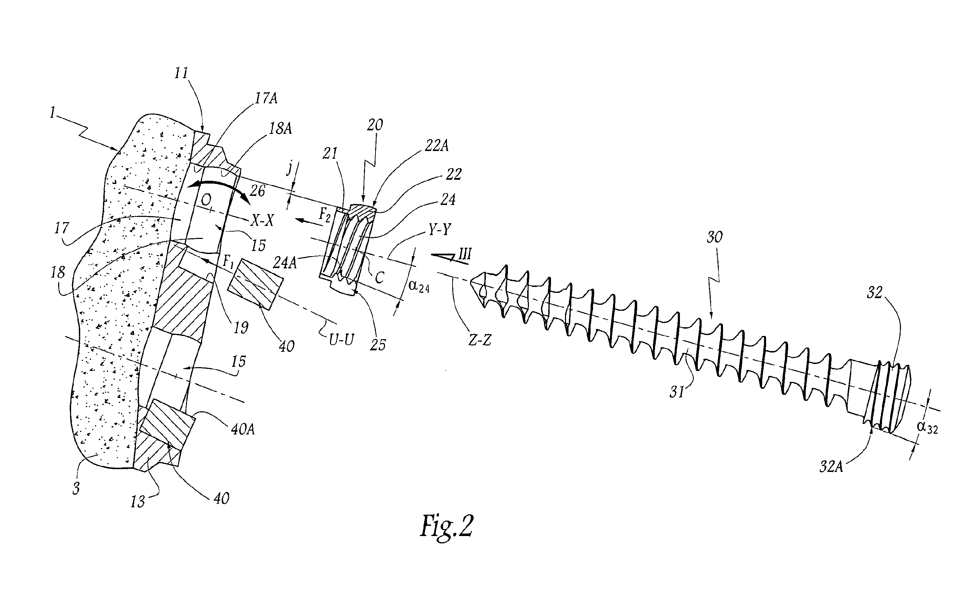

La partie 13 est traversée, dans son épaisseur, de quatre trous 15 débouchant sur l'épiphyse humérale 3 et individuellement identiques les uns aux autres. Les deux trous 15 les plus bas, vus en coupe à la figure 2, sont disposés l'un derrière l'autre suivant la direction longitudinale du corps 11, tandis que les deux trous restants sont disposés, de manière symétrique, de part et d'autre du plan médian de ce corps, qui correspond au plan de coupe II.

La plaque humérale 10 comporte également deux pattes latérales 16 s'étendant de part et d'autre de la partie épiphysaire 13, suivant des directions respectives opposées, transversales à la direction longitudinale de ce corps. En coupe transversale, c'est-à-dire dans un plan de coupe sensiblement perpendiculaire au plan II, les pattes 16 et la partie 13 présentent une forme globale de C dimensionnée pour enserrer l'épiphyse 3, en vue d'améliorer la stabilité de la plaque 10 sur l'humérus 1.

Pour fixer la plaque 10 à l'humérus 1, plusieurs moyens d'ancrage osseux sont destinés à être utilisés, en étant respectivement introduits dans les trous 14 de la partie diaphysaire 12, dans des trous 16a prévus à l'extrémité libre de chaque patte 16, et dans les trous 15 de la partie épiphysaire 13. Seuls les moyens d'ancrage associés aux trous 15 vont être détaillés ci-dessous, étant entendu que les formes des moyens d'ancrage associés aux trous 14 et 16a, non représentés sur les figures, ne sont pas limitatifs de l'invention.

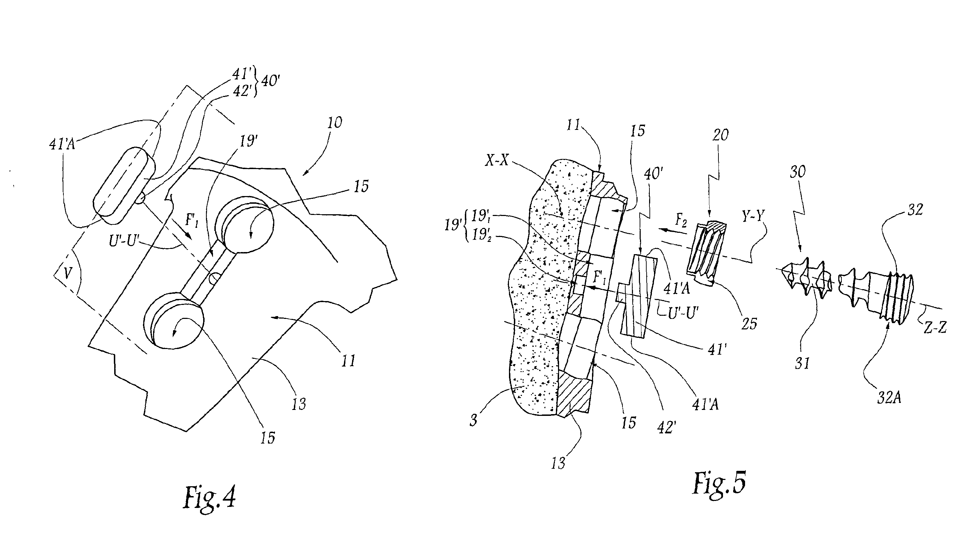

Comme représenté plus en détail aux figures 2 et 3, chaque trou traversant 15 comporte successivement, d'une part, du côté du corps 11 destiné à être tourné vers l'humérus 1, une partie cylindrique 17 à base circulaire et d'axe longitudinal X-X et, d'autre part, du côté opposé, une partie sphérique 18 centrée en un point O appartenant à l'axe X-X. Suivant l'axe X-X, la partie sphérique 18 correspond sensiblement à la zone médiane de la sphère géométrique à laquelle appartient cette partie 18, de sorte que le point O est situé à l'intérieur de la partie 18.

Les axes X-X de chaque trou 15 ne sont pas nécessairement parallèles les uns aux autres, comme représenté pour les deux trous vus en coupe à la figure 2.

Chaque trou 15 est adapté pour recevoir à la fois une olive 20, globalement tubulaire d'axe longitudinal Y-Y, et une vis d'ancrage osseux 30 d'axe longitudinal Z-Z.

Au niveau de son côté distal, c'est-à-dire de son côté destiné à être tourné vers l'humérus 1, l'olive 20 comporte une partie annulaire 21 à base circulaire d'axe Y-Y et, du côté opposé, une partie 22 en forme de bague, à face extérieure 22A sensiblement sphérique centrée en un point C appartenant à l'axe Y-Y. Le diamètre extérieur de la partie annulaire 21 est strictement inférieur au diamètre intérieur de la partie 17 du trou 15, tandis que le diamètre extérieur de la partie 22 est sensiblement égal au diamètre intérieur de la partie 18 du trou. De la sorte, lorsque l'olive 20 est logée dans le trou 15, un jeu radial j non négligeable existe entre les parties 21 et 17, tandis que la face sphérique 22A de la partie 22 et la paroi sphérique 18A de la partie 18 sont accolées de manière complémentaire, les points O et C étant alors sensiblement confondus.

L'olive 20 délimite intérieurement un orifice traversant 24 centré sur l'axe Y-Y et taraudé sur une partie de sa longueur suivant cet axe. L'olive délimite également, sur une portion de sa périphérie, une fente 25 s'étendant sur toute l'olive selon la direction de l'axe Y-Y et qui traverse de part en part la paroi tubulaire de l'olive, de sorte que cette fente relie radialement la face extérieure de l'olive et son orifice intérieur 24. La fente 25 confère ainsi à l'olive une forme globale de C en coupe transversale, ainsi que lorsqu'elle est observée selon l'axe Y-Y, comme à la figure 3.

Suivant sa longueur, la vis 30 comporte, d'une part, une tige distale 31 filetée, destinée à être vissée dans l'épaisseur osseuse de l'épiphyse humérale 3 et, d'autre part, une tête proximale 32 filetée, destinée à être vissée dans l'orifice intérieur 24 de l'olive 20. La tête de vis est adaptée pour permettre l'entraînement en rotation la vis autour de son axe Z-Z, à la fois pour visser sa tige 31 dans l'os huméral et pour visser sa tête 32 dans l'orifice 24 de l'olive. A cet effet, la tête 32 présente par exemple, sur son côté proximal, une cavité, non visible sur les figures, à profil hexagonal ou analogue, permettant l'entraînement en rotation de la vis au moyen d'un outil approprié.

La face extérieure filetée 32A de la tête 32 présente une forme d'enveloppe tronconique d'axe Z-Z et convergent vers cet axe en s'approchant de la tige 31. La paroi 24A de l'orifice taraudé 24 de l'olive 20 présente, elle aussi, une forme d'enveloppe tronconique d'axe Y-Y et convergent vers cet axe en s'approchant du côté distal de l'olive. Les formes tronconiques de la face extérieure 32A et de la paroi 24A sont sensiblement complémentaires, les angles au sommet α₃₂ et α₂₄ de ces deux formes tronconiques étant sensiblement égaux. De plus, le diamètre extérieur de l'extrémité distale de la tête 32 est sensiblement égal au diamètre intérieur de l'extrémité proximale de l'orifice 24, de sorte que, au fur et à mesure du vissage de la tête 32 dans l'olive 20, le diamètre intérieur de l'orifice 24 augmente, jusqu'à ce que le diamètre intérieur de l'extrémité proximale de cet orifice soit sensiblement égal au diamètre extérieur de l'extrémité proximale de la tête 32 lorsque la totalité de cette tête est vissée dans l'olive. L'augmentation du diamètre intérieur de l'orifice 24 est permis par le fait que l'olive 20 est fendue : lors du vissage de la tête 32 dans l'orifice 24, les bords de la fente 25 s'écartent l'un de l'autre, faisant ainsi passer l'olive d'une première configuration, représentée en pointillés à la figure 3 et correspondant à un état au repos de l'olive 20 dans le trou 15, à une seconde configuration déformée radialement vers l'extérieur par rapport à l'axe Y-Y, représentée en traits pleins à la figure 3.

Pour bloquer dans le trou 15 l'olive 20 en rotation autour de l'axe Y-Y lorsqu'elle est dans sa première configuration, un pion 40 est rapporté de manière fixe au corps 11 de la plaque 10. Ce pion se présente sous la forme d'un cylindre à base circulaire, d'axe longitudinal central U-U, réalisé notamment en un métal analogue à celui du corps de plaque 11. Ce pion est assemblé à la plaque 10 en étant reçu et immobilisé, notamment par soudage, dans un logement complémentaire 19 formé, par exemple par usinage, dans le corps de plaque 11, au niveau d'une portion périphérique du trou 15. En pratique, le logement 19 est plus petit que le trou 15, dans le sens où son diamètre est inférieur à celui de ce trou. De plus, le logement 19, dont l'axe longitudinal central peut être, par rapport à l'axe X-X du trou, parallèle ou, comme dans l'exemple considéré aux figures, légèrement incliné de manière convergente vers l'humérus, de préférence sous un angle d'au plus 15°, débouche radialement dans le trou 15. Autrement dit le trou 15 et le logement 19 ne sont pas respectivement fermés sur toute leur périphérie, mais des portions respectives de ces périphéries sont prévues communicantes l'une avec l'autre, en particulier dans le plan de la figure 2. Le logement 19 présente ainsi une forme en portion de cylindre. De la sorte, lorsque le pion 40 est logé dans le logement 19, comme illustré pour le trou 15 représenté dans la partie basse de la figure 2 et comme illustré à la figure 3, ce pion occupe tout le volume interne du logement, tandis qu'une portion périphérique 40A de ce pion occupe une partie de l'intérieur du trou 15 correspondant. Cette portion 40A est prévue pour s'étendre, suivant une direction périphérique à l'axe Y-Y, entre les bords de la fente 25 lorsque l'olive est logée dans le trou 15, comme représenté à la figure 3.

La fabrication et l'utilisation du dispositif d'ostéosynthèse, incluant la plaque 10, l'olive 20, la vis 30 et le pion 40, sont les suivantes :

Initialement, on considère que les quatre pions 40 respectivement associés aux quatre trous 15 ont été assemblés à la plaque 10, en soudant chacun de ces pions dans le logement correspondant 19 préalablement formé dans le corps de plaque 11, comme indiqué par la flèche F₁ à la figure 2. Puis, les olives 20 sont logées dans leur trou 15 associé, avec leur fente 25 chacune positionnée angulairement de telle sorte que la portion périphérique 40A de chaque pion 40 soit reçue entre les bords de cette fente, comme indiqué par la flèche F₂. En pratique, la mise en place de chaque olive 20 nécessite de légèrement comprimer radialement vers l'intérieur la partie extérieurement sphérique 22, en rapprochant un peu l'un de l'autre les bords de sa fente 25, jusqu'à ce que le diamètre extérieur maximal de l'olive soit inférieur au diamètre proximal du trou 15. L'olive peut alors être axialement introduite en totalité dans le trou, avec la portion de pion 40A reçue dans la fente 25. Si besoin, la fente 25 est ainsi partiellement refermée jusqu'à ce que ses bords viennent au contact de la paroi latérale de la portion de pion 40A. La forme cylindrique de cette paroi latérale se révèle pratique pour ne pas gêner la fermeture partielle de la fente 25, voire pour guider l'introduction de l'olive dans le trou, par contact glissant des bords de sa fente le long de cette paroi latérale.

Le chirurgien met ensuite en place la plaque 10 le long de l'humérus 1, en enserrant son épiphyse 3 par la partie 13 du corps 11 et par les pattes 16, comme représenté à la figure 1.

Au niveau de chaque trou 15, la vis 30 est alors axialement introduite, en entraînant cette vis en rotation autour de son axe Z-Z, de sorte que sa tige 31 pénètre dans la matière osseuses de l'épiphyse 3, pour fermement s'y ancrer. L'introduction et la progression de la vis 30 sont réalisées alors que l'olive 20 est logée dans le trou 15, la vis passant à travers son orifice 24. Dans cette configuration, la face sphérique 22A de l'olive glisse librement contre la paroi sphérique 18A du trou 15 pour accommoder un défaut d'alignement entre les axes X-X et Z-Z si le chirurgien introduit la vis 30 suivant une direction longitudinale inclinée par rapport à l'axe du trou, notamment en fonction de l'état de la matière osseuse à sa disposition. La liaison mobile entre l'olive et la paroi du trou 15 s'apparente alors à celle d'une rotule, avec cependant une liberté de mouvement restreinte par la présence du pion 40. En effet, comme la portion de pion 40A est reçue dans la fente 25, l'olive 20 est empêchée de tourner en rotation sur elle même, c'est à dire en rotation autour de son axe Y-Y, de sorte qu'elle n'est mobile, par rapport au corps 11, que sensiblement dans un plan correspondant au plan de coupe de la figure 2. Autrement dit, pour accommoder le désalignement potentiel des axes X-X et Z-Z, l'olive 20 est à même de pivoter, à l'intérieur du trou 15, autour d'un axe sensiblement perpendiculaire à l'axe X-X et passant par le point O, par contact glissant de la face 22A contre la paroi 18A, comme indiqué par la flèche 26 à la figure 2. L'amplitude de ce pivotement est limitée par la mise en butée de la partie annulaire 21 contre la paroi 17A de la partie de trou 17. Autrement dit, l'amplitude maximale du pivotement de l'olive est directement liée au jeu j précité et vaut, en pratique, une vingtaine de degrés.

Lorsque la tête 32 est au voisinage axial immédiat de l'olive 20 et que le chirurgien continue d'entraîner en rotation la vis 30 autour de son axe Z-Z, le filet de la face extérieure 32A de cette tête s'engage facilement et aisément dans le taraudage de l'orifice 24, faisant passer l'olive de sa première à sa seconde configuration. L'amorce de la mise en prise du filet de la tête de vis dans le taraudage interne de l'olive est facilitée par l'immobilisation en rotation autour de son axe Y-Y de l'olive 20, par le pion 40.

Au fur et à mesure que la tête de vis 32 est vissée dans l'orifice taraudé 24, la face extérieure 22A de l'olive 20 est pressée contre la paroi 18A du trou 15, jusqu'à coincer l'olive dans le trou, liant alors rigidement l'olive, et donc la vis 30, à la plaque 10.

Sur les figures 4 et 5 est représentée une variante de réalisation du dispositif d'ostéosynthèse des figures 1 à 3, les composants communs à ces deux possibilités de réalisation portant les mêmes références numériques. La variante des figures 4 et 5 se distingue du dispositif des figures 1 à 3 par essentiellement la forme d'au moins un de ses pions 40' de blocage en rotation des olives 20 dans les trous 15. Plus précisément, plutôt que d'associer à chaque trou 15 l'un des pions cylindriques 40 du dispositif des figures 1 à 3, le même pion 40' permet de bloquer en rotation deux olives 20 logées dans deux des trous 15 adjacents, par exemple dans les deux trous 15 les plus bas pour la plaque humérale 10, comme illustré sur les figures 4 et 5. Bien entendu, un pion du même type que le pion 40' décrit ci-après pourrait, en variante non représentée, être utilisé pour bloquer les olives 20 logées dans les deux trous 15 situés dans la partie haute du corps de plaque 11, de part et d'autre du plan médian de ce corps.

Comme représenté sur les figures 4 et 5, le pion 40' comprend une partie proximale 41' de forme globalement parallélépipédique et une partie distale 42' de forme globalement cylindrique à base circulaire, centrée sur un axe U'-U' perpendiculaire à la dimension longitudinale de la partie 41'. Dans le plan de la figure 5, le pion 40' présente ainsi une forme globale de T.

Pour recevoir et immobiliser ce pion 40', le corps de plaque 11 délimite un logement 19' qui inclut, du côté proximal du corps de plaque, une partie allongée 19'₁ qui s'étend en longueur entre les deux trous 15, dans le plan passant par les axes X-X de ces trous, c'est-à-dire suivant globalement une direction radiale à ces trous. Les extrémités longitudinales de cette partie de logement 19'₁ débouchent respectivement dans les deux trous 15.

Du côté distal du corps de plaque 11, le logement 19' inclut une partie cylindrique 19'₂ débouchant dans la partie de logement 19'₁, de telle sorte que les parties de logement 19'₁ et 19'₂ sont respectivement complémentaires des parties proximale 41' et distale 42' du pion 40'.

Pour rapporter le pion 40' dans le logement 19', la partie distale 42' est introduite de manière co-axiale dans la partie de logement 19'₂, comme indiqué par la flèche F'₁, tandis que la partie proximale 41' est introduite de manière ajustée dans la partie de logement 19'₂, empêchant ainsi toute rotation du pion 40' autour de l'axe U'-U'. Les extrémités longitudinales 41'A de la partie proximale 41' occupent alors une partie de chacun des trous 15, de la même façon que la portion périphérique 40A du pion 40 occupe une partie du trou 15 pour le mode de réalisation des figures 1 à 3. Avantageusement, ces extrémités longitudinales 41'A sont conformées en des portions de cylindre dont les axes longitudinaux respectifs appartiennent au plan de la figure 5, afin de faciliter la mise en place des olives 20 dans les trous 15.

La fabrication et l'utilisation de la variante des figures 4 et 5 sont analogues à celles du dispositif envisagé aux figures 1 à 3 : après avoir placé et bloqué par complémentarité de formes le pion 40' dans le logement 19', les olives 20 sont chacune introduites dans l'un des trous 15, avec les extrémités 41'A du pion 40' reçues entre les bords de la fente 25 de chaque olive, comme indiqué par la flèche F₂. Les vis 30 sont ensuite axialement introduites dans chaque trou 15, comme décrit plus haut pour la forme de réalisation des figures 1 à 3.

Divers aménagements et variantes aux dispositifs d'ostéosynthèse décrits ci-dessus sont en outre envisageables :
- de manière générale, l'agencement trou 15/olive 20/vis 30 peut être prévu au niveau d'un corps d'une prothèse, en vue de fixer ce corps à un os à traiter ;
- d'autres moyens d'ancrage osseux que la vis 30 décrite ci-dessus sont possibles, par exemple des broches, des clous ou analogues, dont une partie proximale est à visser dans une olive bloquée en rotation autour de son axe, comme l'olive 20 ;
- le nombre, la disposition ainsi que les dimensions des trous 15 de réception des olives et des moyens d'ancrage osseux ne sont pas limités à ceux décrits ci-dessus, mais sont adaptables au corps prothétique ou d'ostéosynthèse à fixer à un os ; et/ou
- d'autres formes que des portions de cylindre peuvent être envisagées pour les parties 40A et 41'A des pions 40 et 40' situées entre les bords de la fente 25 des olives 20, étant remarqué que, avantageusement, ces parties de pion se rétrécissent en direction du centre du trou 15 qu'elles occupent en partie, afin de ne pas gêner la fermeture partielle de la fente lors de l'introduction de l'olive dans le trou, tout en étant ajustées au plus près des bords de cette fente une fois que l'olive est logée dans ce trou.

## Revendications

1. Dispositif prothétique ou d'ostéosynthèse, comportant :
- un corps prothétique ou d'ostéosynthèse (11) délimitant au moins un trou traversant (15) de réception d'un moyen allongé d'ancrage osseux (30),
- au moins une olive (20) de liaison mécanique entre le corps et le moyen d'ancrage, ladite olive délimitant, à la fois, un orifice intérieur (24) de vissage d'une partie proximale (32) du moyen d'ancrage et une fente (25) qui traverse l'olive de part en part entre sa face extérieure et son orifice, et étant adaptée, lors du vissage de la partie proximale du moyen d'ancrage, pour passer, par écartement relatif des bords de sa fente, d'une première configuration dans laquelle l'olive est logée de manière mobile dans le trou, à une seconde configuration dans laquelle l'olive relie rigidement le moyen d'ancrage et le corps par coincement entre la partie proximale de ce moyen et une paroi (18A) du trou, et
- un moyen de blocage (40 ; 40') adapté pour, lorsque l'olive (20) est dans sa première configuration, bloquer l'olive en rotation autour de l'axe (Y-Y) de son orifice (24) par rapport au corps (11), ce moyen de blocage étant solidaire du corps et s'étendant au moins partiellement à l'intérieur du trou de manière à être reçu entre les bords de la fente (25) de l'olive,
**caractérisé en ce que** le moyen de blocage comporte un pion (40 ; 40') rapporté de manière fixe dans un logement (19 ; 19') délimité par le corps (11) et débouchant dans le trou (15), le pion occupant de manière continue le logement et une partie du trou.

2. Dispositif suivant la revendication 1, **caractérisé en ce que** le pion (40) est globalement cylindrique et le logement (19) présente une forme en portion de cylindre dont la section transversale est sensiblement complémentaire à celle du pion et dont l'axe central est, par rapport à l'axe central (X-X) du trou (15), parallèle ou incliné sous un angle d'au plus 15°.

3. Dispositif suivant la revendication 1, **caractérisé en ce que** le logement (19') présente au moins une partie (19'₁) de forme allongée, qui s'étend en longueur suivant une direction sensiblement radiale au trou (15), qui relie ce trou à un autre trou traversant (15) du corps (11) destiné à recevoir un autre moyen d'ancrage osseux (30) avec interposition d'une autre olive fendue (20), et qui reçoit de manière complémentaire une partie allongée (41') du pion (40'), dont les extrémités longitudinales (41'A) sont respectivement reçues dans la fente (25) des olives (20) respectivement associées aux deux trous (15) reliés par le logement.

4. Dispositif suivant l'une quelconque des revendications précédentes, **caractérisé en ce que** la ou chaque partie (40A ; 41'A) du moyen de blocage (40 ; 40') située entre les bords de la fente (25) de la ou chaque olive (20) se rétrécit en direction de l'intérieur du trou (15).

5. Dispositif suivant l'une quelconque des revendications précédentes, **caractérisé en ce que** la face extérieure (22A) de la ou chaque olive (20) et la paroi (18A) du ou de chaque trou (15), excepté respectivement au niveau de la fente (25) et au niveau du moyen de blocage (40 ; 40'), sont sensiblement sphériques de manière sensiblement complémentaire.

6. Dispositif suivant l'une quelconque des revendications précédentes, **caractérisé en ce que** la paroi (24A) de l'orifice (24) de la ou chaque olive (20) et la face extérieure (32A) de la partie proximale (32) du ou de chaque moyen d'ancrage (30) sont globalement tronconiques de manière sensiblement complémentaire.

7. Dispositif suivant l'une quelconque des revendications précédentes, **caractérisé en ce que** le ou chaque moyen d'ancrage constitue, au moins en partie, une vis (30) dont la tête (32) d'entraînement en rotation correspond à ladite partie proximale, et **en ce que** le corps (11) constitue, au moins en partie, une plaque humérale (10).

8. Procédé de fabrication d'un dispositif prothétique ou d'ostéosynthèse, dans lequel on dispose :
- d'un corps prothétique ou d'ostéosynthèse (11) délimitant au moins un trou traversant (15) de réception d'un moyen allongé d'ancrage osseux (30), et
- d'au moins une olive (20) de liaison mécanique entre le corps et le moyen d'ancrage, ladite olive délimitant, à la fois, un orifice intérieur (24) de vissage d'une partie proximale (32) du moyen d'ancrage et une fente (25) qui traverse l'olive de part en part entre sa face extérieure et son orifice, et étant adaptée, lors du vissage de la partie proximale du moyen d'ancrage, pour passer, par écartement relatif des bords de sa fente, d'une première configuration dans laquelle l'olive est logée de manière mobile dans le trou, à une seconde configuration dans laquelle l'olive relie rigidement le moyen d'ancrage et le corps par coincement entre la partie proximale de ce moyen et une paroi (18A) du trou,
**caractérisé en ce qu'**on forme dans le corps (11) un logement (19 ; 19'), qui est distinct du trou (15) et qui débouche dans le trou, et, dans ce logement, on rapporte de manière fixe un pion (40 ; 40') qui occupe de manière continue le logement et une partie du trou de telle sorte qu'une partie (40A ; 41'A) de ce pion est reçue entre les bords de la fente (25) de l'olive (20) lorsque cette olive est dans sa première configuration, bloquant ainsi l'olive en rotation autour de l'axe (Y-Y) de son orifice (24) par rapport au corps (11).

9. Procédé suivant la revendication 8, **caractérisé en ce que** le pion (40 ; 40') est rapporté de manière fixe dans le logement (19 ; 19') par soudage et/ou par complémentarité de formes.

10. Procédé suivant l'une des revendications 8 ou 9, **caractérisé en ce que** le pion (40) est globalement cylindrique et **en ce qu'**on donne au logement (19) une forme en portion de cylindre dont la section transversale est sensiblement complémentaire à celle du pion et dont l'axe central est, par rapport à l'axe central (X-X) du trou (15), parallèle ou incliné sous un angle d'au plus 15°.

11. Procédé suivant l'une des revendications 8 ou 9, **caractérisé en ce qu'**on donne à au moins une partie (19'₁) du logement (19') une forme allongée, qui s'étend en longueur suivant une direction sensiblement radiale au trou (15), qui relie ce trou à un autre trou traversant (15) du corps (11) destiné à recevoir un autre moyen d'ancrage osseux (30) avec interposition d'une autre olive fendue (20), et qui reçoit de manière complémentaire une partie allongée (41') du pion (40'), dont les extrémités longitudinales (41'A) sont respectivement reçues dans la fente (25) des olives (20) respectivement associées aux deux trous (15) reliés par le logement.
